# EUROPÄISCHE PATENTSCHRIFT

(11) **EP 3 888 710 B1**
(45) Veröffentlichungstag und Bekanntmachung des Hinweises auf die Patenterteilung: **23.08.2023**
(21) Anmeldenummer: 20177765.3
(22) Anmeldetag: 02.06.2020
(51) Int. Cl.: A61L 27/24, A61L 27/54, A61L 27/22, A61L 31/04, A61L 31/16

(54) **VERFAHREN ZUM AUFBEREITEN VON KOLLAGENFORMKÖRPERN SOWIE KOLLAGENFORMKÖRPERN**
COLLAGEN MOULDED BODIES AND METHOD FOR PREPARING COLLAGEN MOULDED BODIES
PROCÉDÉ DE TRAITEMENT DES CORPS MOULÉS DE COLLAGÈNE ET CORPS MOULÉS DE COLLAGÈNE

(30) Priorität: 01.04.2020 DE 102020108991
(43) Veröffentlichungstag der Anmeldung: 06.10.2021
(73) Patentinhaber: Kasperk, Christian, 69123 Heidelberg (DE); Haas, Andreas, 69126 Heidelberg (DE)
(72) Erfinder: HAAS, Andreas, 69126 Heidelberg (DE)
(74) Vertreter: Wunderlich & Heim Patentanwälte Partnerschaftsgesellschaft mbB

(56) Entgegenhaltungen:
- EP-A1- 1 283 063
- JP-A- H02 156 954
- AMARJOT SINGH ET AL: "Role of osteopontin in bone remodeling and orthodontic tooth movement: a review", PROGRESS IN ORTHODONTICS, BIOMED CENTRAL LTD, LONDON, UK, Bd. 19, Nr. 1, 25. Juni 2018 (2018-06-25), Seiten 1-8, XP021257718, DOI: 10.1186/S40510-018-0216-2

## Beschreibung

Die Erfindung betrifft ein Verfahren zum Aufbereiten von Kollagenformkörpern, bei dem Kollagenformkörper aus Kollagen Typ I und/oder Kollagen Typ III verwendet werden. Kollagenformkörper können auch als Kollagenmatrix bezeichnet werden. Ferner betrifft die Erfindung Kollagenformkörper, welche Kollagen Typ I und/oder Kollagen Typ III aufweisen.

Im Rahmen von Operationen zum Einbringen von Zahnimplantaten in den Kieferknochen werden oft Knochenaufbauten, sogenannte Kieferknochenaugmentationen, benötigt, da das Knochenangebot für eine Verankerung im zahnlosen Kieferabschnitt aufgrund von Knochenatrophie nach einem Zahnverlust meist nicht ausreicht. Das hierfür eingebrachte Knochenersatzmaterial, in welches neuer Knochen einwachsen soll, nimmt ein nicht zu vernachlässigendes Volumen ein. Daher reicht oftmals die vorhandene Mundschleimhaut nicht ganz aus, um den Defekt beziehungsweise das Knochenersatzmaterial sicher und spannungsfrei abzudecken. So kommen oftmals industriell hergestellte Membranen, meist aus Kollagen gefertigt, zum Einsatz, um den Defekt zu überdecken.

Aber auch wenn der Defekt und damit der Knochenaufbau gegenüber der Mundhöhle sicher abgedeckt werden kann, sollte der Knochenaufbau zur Einheilung räumlich von der aufliegenden und bedeckenden Schleimhaut getrennt werden, damit ein Einwachsen des sich wesentlich schneller vermehrenden Bindegewebes der Schleimhaut in das eingebrachte Knochenaugmentationsmaterial verhindert werden kann und der Knochen bei Einwachsen in das augmentierte Material nicht behindert wird.

Wünschenswert ist es auch, wenn durch einen Wundverschluss auch das Eindringen von Bakterien in die Wunde und damit eine Infektion des augmentierten Knochenmaterials vermieden werden. Auch sollte durch das Bedecken des Augmentationsmaterials dieses an Ort und Stelle gehalten und verhindert werden, dass dieses durch den Speichel ausgewaschen wird. Auch hierzu dienen die eingebrachten handelsüblichen Membranen.

Ein weiterer Wunsch ist es, dass die Membran das eingebrachte Knochenersatzmaterial möglichst formstabil an Ort und Stelle hält und idealerweise verhindert, dass das Augmentat in gewisser Weise mechanisch durch Beanspruchung "abrutscht". Die Membran soll somit auch einen stabilisierenden Effekt haben.

Industriell gefertigte Membranen werden hierzu neuerdings mit Titan verstärkt. Diese müssen jedoch nach Heilung des Knochens in einem Zweiteingriff entfernt werden. Aufgrund seiner vollständigen Degradation in wässrigem Milieu sind derzeit magnesiumverstärkte und damit vollständig resorbierbare Membranen in Erprobung, welche dann einen solchen Zweiteingriff zur Entfernung des Metalls ersparen sollen. Auch formende Gitter aus Titan, welche dann ebenso wieder in einem Zweiteingriff entfernt werden müssen oder entnommener körpereigener Knochen des Patienten oder Spenderknochen werden als eine Art "Spundwand" an den Kieferknochen gelehnt oder mit diesem verschraubt, um eine Stabilisierung des Knochenersatzmaterials auf dem Kieferkamm zu gewährleisten. Der dabei entstandene Hohl- beziehungsweise Freiraum kann dann letztlich mit autologem, allogenem, xenogenem Knochen und/oder synthetischem Knochenersatzmaterial aufgefüllt werden.

Ferner sollte durch den Verschluss der Wunde, also des offenen Implantatbettes sowie der angrenzenden Bereiche durch derartige Membranen eine verbesserte Einheilung des Knochenersatzmaterials erreicht werden, indem auf der defektseitigen Membranfläche die Adhärenz also eine Verankerung und dadurch das Aufwachsen von Knochenzellen befördert wird. Dadurch sollte die knöcherne Durchbauung des Defektes begünstigt werden. Auch wäre wünschenswert, wenn eine solche Membran dabei zusätzlich durch Versteifung einen stabilisierenden Charakter hätte, was das Augmentat vor mechanischen Belastungen orts- und formstabil hält.

Die aktuell im Handel erhältlichen Membranen ermöglichen bisher lediglich einen spannungsfreien Wundverschluss, bilden eine physiologische Barriere zur Verhinderung des Einwachsens von weichem Bindegewebe in das Knochenersatzmaterial und hierdurch bedingter Störung der stabilen Verknöcherung aus und haben teilweise formgebenden Charakter und schützen so vor mechanischen Belastungen und Kräften, sofern es sich um verstärkte Membranen handelt. Sie dienen zusammenfassend somit lediglich dem Wundverschluss und einer räumlichen Trennung von Knochenaugmentat und Schleimhaut. Sie besitzen allerdings keinerlei Knochenwachstum zusätzlich begünstigende Eigenschaften, nur rudimentäre formstabilisierende Eigenschaften sowie nur geringen mechanischen Schutz vor äußeren mechanischen Einflüssen.

Weiter werden bei geplanter Implantation von Zahnimplantaten nach der Extraktion des Zahnes im Rahmen von einer sogenannten Socket- / Ridge Preservation Formkörper aus Kollagen in das leerer Zahnfach eingebracht. Grund hierfür ist die Tatsache, dass nach einer Zahnextraktion der vom Körper nicht mehr benötigte Knochen an der Stelle des nun fehlenden Zahnes physiologischerweise muldenförmig abgebaut wird. Somit steht dann deutlich weniger Knochen für eine Implantation nach Ausheilung der Extraktionswunde zur Verfügung und ein aufwändiger Knochenaufbau wird vor Implantation nötig. Um diesen Knochenabbau nach Zahnextraktion zu minimieren, kommt die Sogenannte Socket- / Ridge Preservation zum Einsatz. Der dabei verwendete Kollagenformkörper dient hierbei dazu, den Blutkoagel stabil an Ort und Stelle zu halten, ein Ausschwemmen zu verhindern und somit eine Art Baugerüst darzustellen, an dem sich schnell neuer Knochen von einer Knochenwand zur anderen bilden und somit einem muldenförmigen Knochenabbau entgegengewirkt wird.

Bisherige Kollagenformkörper stellen jedoch nur eine Leitschiene für das Knochenwachstum dar. Somit verläuft die Konchenheilung langsam und eine gewisse Mulde mit Knochenschwund bildet sich trotzdem aus. Auch tragen Infektionen in der Mundhöhle dazu bei, dass immer mit einem gewissen entzündungsbedingten Knochenschwund durch entzündlich behinderte Knochenbildung und insbesondere entzündlich bedingter Induktion des Knochenabbaus zu rechnen ist.

Aus der EP 1 283 063 ist eine Kollagenmembran bekannt, welche Kollagen I, Kollagen III oder Kombinationen davon aufweist. Zusätzlich ist die Kollagenmembran mit Nucleinsäuresequenzen beladen, welche ein Wachstum von Knochen und/oder Körperzellen fördern sollen.

Der Erfindung liegt daher die **Aufgabe** zugrunde, ein Verfahren zum Aufbereiten von Kollagenformkörpern und Formkörpern aus Kollagen anzugeben, welche das Knochenzellwachstum positiv beeinflussen.

Diese Aufgabe wird erfindungsgemäß durch ein Verfahren zum Aufbereiten von Kollagenformkörpern mit den Merkmalen des Anspruchs 1 sowie mit einem Kollagenformkörper mit den Merkmalen des Anspruchs 8 gelöst. Vorteilhafte Ausführungsformen der Erfindung sind in den Unteransprüchen angegeben.

Entsprechend der Erfindung ist vorgesehen, dass auf Ausgangskollagenformkörpern, welche im Wesentlichen aus Kollagen Typ I und/oder Typ III bestehen, Knochenmatrixproteine in einer Flüssigkeit auf den Kollagenformkörper aufgebracht werden. Hierbei ist die Menge der Flüssigkeit derart bemessen, dass die Knochenmatrixproteine, welche sich in der Flüssigkeit befinden, zwischen 0,01 Mass.-% bis 5 Mass.-% der Masse des Kollagenformkörpers ausmachen.

Der Erfindung liegt die Idee zugrunde, Knochenmatrixproteine zu verwenden, um Kollagenformkörpern positive Eigenschaften bezüglich des Knochenwachstums zu verleihen. Dabei wurde erkannt, dass es ausreicht, die Knochenmatrixproteine, welche sich in einer Flüssigkeit befinden, auf den Formkörper aufzutropfen. Durch die vorhandenen Van-der-Waals-Kräfte zwischen den Proteinmolekülen bleiben die aufgetropften Knochenmatrixmoleküle in dem Kollagenformkörper beziehungsweise der Kollagenmatrix des Formkörpers haften. Nach einem anschließenden Trocknen bleiben sie so letztendlich in der Kollagenmatrix eingebettet.

Auf diese Weise kann eine natürliche Knochengewebematrix, welche aus ca. 90% Kollagen und zu etwa 10% aus Knochenmatrixmolekülen besteht, nachgebildet oder besser nachempfunden werden.

Grundsätzlich kann ein Kollagenformkörpern eine beliebige geometrische Form haben. Es ist so beispielsweise möglich ihn zylindrisch oder kegelförmig auszubilden. In diesem Sinne der Erfindung kann ein Kollagenformkörpern demnach auch in Form einer Kollagenmembran ausgebildet werden. Andes ausgedrückt, ist in diesem Sinne eine Membran ein entsprechende flach und blattartig ausgebildeter Formkörper.

Im Folgenden wird der grundlegende Zusammenhang zwischen Knochenmatrixmolekülen und dem vermehrten Knochenaufbau näher beschrieben.

Grundsätzlich besteht jedes menschliche oder tierische Gewebe aus einer Matrix in der sich die Gewebezellen verankern können. Beispielsweise besteht Knochengewebe aus anorganischem Hydroxylapatit und einer organischen Knochengewebematrix. Die organische Knochengewebsmatrix besteht zu etwa 90 % aus Kollagen und zu etwa 10 % aus weiteren Knochenmatrixmolekülen.

Mesenchymale Zellen, wie Knochenzellen, aber auch epidermale Zellen, wie Schleimhautzellen, verankern sich mit extrazellulären Umgebungsmatrizes über Integrine in der Zellmembran. Integrine sind Oberflächenrezeptoren in der Zellmembran von Gewebezellen, die sich an die Umgebungsmatrix anheften. Dadurch sitzt die Zelle fest in dem zellulären Gewebeverbund beispielsweise des Knochengewebes oder im Verbund der Mundschleimhaut.

Durch den Kontakt der zellulären Integrine mit der Umgebungsmatrix nimmt die Zelle direkt Verformungen der Umgebungsmatrix war, wie biomechanische Belastung des Knochengewebes oder Dehnung an der Mundschleimhaut. Diese "Zugwirkung" an den zellulären Integrinen aktiviert durch die Verknüpfung der Integrine mit dem intrazellulären Zytoskelett intrazellulär Signalketten über beispielsweise sogenannte "focal adhesion kinase", was schließlich zu zellulären Reaktionen führt.

Diese zellulären Reaktionen können Zellteilungen, vermehrte Bildung von Matrixmolekülen wie Kollagen oder auch die verbesserte Verankerung der Zelle im Gewebeverbund sein. Im Knochengewebe wird so beispielsweise belastungsabhängig Knochengewebsneubildung stimuliert oder Schleimhautzellen verankern sich fester in der Matrix der Mundschleimhaut.

Es gibt eine große Zahl verschiedener Integrine an der Zellmembranoberfläche von Knochen- und Schleimhautzellen, die bevorzugt an bestimmte Epitope der Matrixmoleküle andocken. Schon alleine die Bindung eines Integrins an das betreffende Epitop eines Umgebungsmatrix-Moleküls signalisiert der Zelle, dass sie ihre zellulären differenzierten Funktionen wie beispielweise Knochenmatrixbildung vollumfänglich aufnehmen kann, da sich die Zelle in der adäquaten Matrixumgebung befindet.

Verschiedene Matrixmoleküle, an denen die Knochenzellen andocken, besitzen also unterschiedliche Epitope, das heißt Erkennungssequenzen, die das Andocken unterschiedlicher Integrine ermöglichen. Neben dem Typ I Kollagen ist zum Beispiel das Fibronektin ein ubiquitär in den Bindegeweben und damit auch in der organischen Knochenmatrix vorkommendes Molekül, an das insbesondere auch Knochen- und Schleimhautzellen über das alpha5-β1-Integrin, welches auch als Fibronektinrezeptor bezeichnet wird, andocken können. Das Andocken der zellmembranständigen Rezeptoren an das in der Umgebungsmatrix der Zellen enthaltene Matrixprotein beispielweise an Fibronektin oder an Bone Sialoprotein bedeutet eine besonders intensive Stimulation des Zellstoffwechsels.

Entsprechend der Erfindung wird vorgeschlagen, insbesondere Osteopontin und/oder Bone Sialoprotein als Knochenmatrixprotein hinzuzufügen, die auch in der reifen organischen humanen Knochenmatrix in vivo so vorliegen. Dadurch wird der Stoffwechsel der sich anheftenden Knochenzellen defektseitig zur vermehrten alkalische Phosphatase Produktion oder zur vermehrten Mineralbildung stimuliert.

Es ist möglich, dass sich die gesamte Menge an Protein, die auf die Membran oder dem Formkörper aufgebracht werden soll, in den wenigen, beispielsweise 4 bis 5, Tropfen Flüssigkeit befindet, die mittels Pipette auf die Membran oder den Formkörper aufgebracht werden. Die Flüssigkeit dient dabei lediglich als Trägerflüssigkeit zur gleichmäßigen Verteilung auf dem bemessenen Membranstück, zum Beispiel 1,5 x 1,5 cm oder einem beliebigen Formkörper, zum Beispiel einem zylindrischen Formkörper mit den Maßen 1,5 cm Länge x 0,5 cm Durchmesser. Die Flüssigkeitsmenge wird dabei so bemessen, dass die gesamte Flüssigkeitsmenge gerade von der Membran oder dem Formkörper aufgesaugt wird.

In einer bevorzugten Ausführungsform befindet sich in der Flüssigkeit zwischen 0,1 Mass.-% und 1 Mass.-% der Masse des Kollagenformkörpers an Knochenmatrixproteinen. Eine derartige Verteilung ergibt sehr gute Ergebnisse.

Um der dem Kollagenformkörper zusätzlich antibakterielle Eigenschaften zu verleihen, werden die Prolinreste des Kollagens des Kollagenformkörpers jodiert. Dazu wird der Kollagenformkörper in ein Medium, welches bevorzugt einen pH-Wert zwischen 5,0 und 6,0 aufweist, eingebracht.

Zusätzlich wird noch Natriumjodid sowie Wasserstoffperoxid hinzugefügt. Anschließend wird die Kollagenmatrix in diesem Medium mit Lactoperoxidase versetzt. Dies führt dazu, dass ca. 2% der Prolinreste des Kollagenformkörpers jodiert werden. Anschließend wird der Kollagenformkörper gereinigt, um so nicht gebundenes Jod zu entfernen. Dieser Schritt des Jodierens kann sowohl vor als auch nach dem Aufbringen der Knochenmatrixproteine erfolgen. Idealerweise liegt der pH-Wert um 5,6. Das Jodieren wird bevorzugt bei einer Temperatur um 25°C durchgeführt.

Durch die so vorhandene antibakterielle Wirkung des Jods wird das Risiko von lokalen Wundinfektionen beim Einbringen der so aufgearbeiteten Kollagenformkörper wie beispielweise Kollagenmembranen verringert.

Beispielsweise könnten bei der zuvor beschriebenen Socket- / Ridge Preservation mit obigem Verfahren jodierte Kollagenzylinder klinische Vorteile aufweisen. So könnte ein entzündlich stimulierter Knochenabbau verhindert werden. Ebenso ist eine antibakterielle Wirkung somit gerade bei Exposition der Wunde in die Mundhöhle gegeben.

Wie zuvor ausgeführt, ist es wünschenswert, wenn im Speziellen Kollagenformkörper wie insbesondere Kollagenmembranen formstabil sind beziehungsweise einen formgebenden Charakter haben. Hierzu können die Formkörper, vor oder nach der oben beschriebenen Aufarbeitung mittels Osteopontin, Bone Sialoprotein und/oder Jodierung der Prolinreste, in oder mit einem Knochenzement, insbesondere in einem Hydroxylapatit-Knochenzement getränkt werden. Anschließend werden die Kollagenformkörper getrocknet. Alternativ oder fakultativ hierzu ist es möglich, die Kollagenformkörper mit einem Knochenzement zu bestreichen. Dies bedeutet, dass der Knochenzement lediglich auf die Oberfläche aufgebracht wird. Auch hierbei werden die Kollagenformkörper anschließend wiederum getrocknet.

Durch die Kombination von Kollagenformkörpern wie Kollagenmembranen mit Knochenzement wird beim Verwenden der Kollagenmembranen eine stabilisierende Wirkung erzielt. So kann ein Knochenaugmentat, welches mit den erfindungsgemäßen Kollagenmembranen abgedeckt wird, zusätzlich stabilisiert und geschützt werden.

In einer weiteren Ausgestaltung der Erfindung kann der Knochenzement, insbesondere der Hydroxylapatit-Knochenzement, vor dem Tränken oder Aufbringen mit Strontium versetzt werden. Hierdurch wird dem Knochenzement und damit auch dem Kollagenformkörper osteoinduktive und/oder antiresorptive Eigenschaften verliehen. Auch dies unterstützt den Knochenheilungsprozess.

Die Dauer der Standfestigkeit und Resorptionszeit des Knochenzementes kann grundsätzlich über den Anteil von Trikalziumphosphat im Zement gesteuert werden.

Um einem Kollagenformkörper wie der Melmbran noch bessere Eigenschaften für das Einheilen des Augmentats zu verleihen, kann der Knochenzement zusätzlich mit Kollagenfasern versetzt werden. Die Fasern können gleiche aber auch unterschiedliche Längen aufweisen. Diese Kollagenfasern können ferner mit Knochenmatrixmolekülen angereichert sein. Das Vorsehen von Kollagenfasern in dem Knochenzement verleiht diesem Elastizität und macht ihn so gegenüber Scher- und Biegebelastungen stabiler.

Die mit dem Knochenzement versetzten Kollagenmembranen können in Art und Weise von Spundwänden eingesetzt werden, die ähnliche Einsatzmöglichkeiten haben wie sie aus Eigenknochen oder auch lyophilisierten Spenderknochen aus einer Spenderbank gefertigte Knochenplättchen oder Knochenchips aufweisen, die ebenfalls bei der Kieferaugmentation verwendet werden.

Des Weiteren betrifft die Erfindung einen Kollagenformkörper, welche hauptsächlich Kollagen des Typs I und/oder des Types III aufweist. Der Kollagenformkörper ist dadurch gekennzeichnet, dass 0,1 Mass.-% bis 5 Mass.-% Kochenmatrixproteine, insbesondere Osteopontin und/oder Bone Sialoprotein, in der Kollagenmatrix vorhanden sind, dass etwa 2% der Prolinreste des Kollagens des Kollagenformkörpers jodiert sind, wobei der Kollagenformkörper zum Jodieren der Prolinreste in ein Medium, welches einen pH-Wert zwischen 5,0 und 6,0 bevorzugt um 5,6 hat und Natriumjodid sowie Wasserstoffperoxid aufweist und dort mit Lactoperoxidase versetzt wird, und der Kollagenformkörper anschließend von nicht gebundenem Jod gereinigt wird.

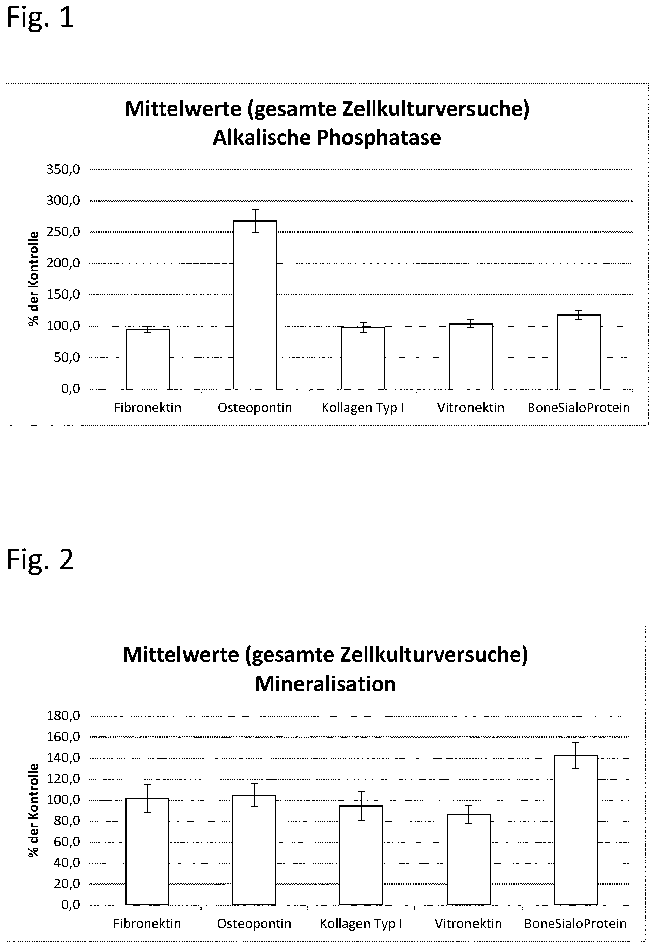

Der Kollagenformkörper kann in Form einer Membran ausgebildet sein. Im Folgenden werden Versuche beschrieben, um die positive Einwirkung von Knochenmatrixproteinen auf den Knochenaufbau näher zu erläutern, wobei Figuren 1 und 2 Ergebnisse der Versuche graphisch darstellen.

### Alkalische Phosphatasenaktivität

In der ersten Versuchsreihe wurde die Auswirkung verschiedener Knochenmatrixmoleküle auf den Knochenzellstoffwechsel anhand der alkalischen Phosphataseaktivität bewertet.

Alkalische Phosphatase ist ein für die Mineral- und Knochenbildung menschlicher Knochenzellen unverzichtbares Enzyms, welches auch als wichtigster Knochenanbaumarker angesehen wird. Alkalische Phosphatase wird von Knochenzellen in das jeweilige Mikromilieu abgegeben, um dort die Mineralbildung zu initiieren.

Diese Untersuchungen fanden in Zellkulturüberständen nach 24 Stunden Zellinkubation auf beschichteten Kulturplatten mit den unten aufgeführten verschiedenen Knochenmatrixporteinen statt. Die Kulturplatten wurden hierbei jeweils 24 Stunden mit 1 yg/ml PBS (Phosphat gepufferter Salzlösung, phosphate-buffered saline) und dem jeweiligen Knochenmatrixmolekül beschichtet.

Die Ergebnisse werden in Prozent der Kontrolle +/- Standardabweichung ausgedrückt. Bei der Kontrolle handelt es sich jeweils um die entsprechenden Zellen, die allerdings in Kulturschalen ohne vorherige Beschichtung mit Knochenmatrixmolekülen kultiviert wurden.

| **1. Versuch (menschliche Unterkieferknochenzellen)** | | |
|---|---|---|
| | % Kontrolle | STA+/- |
| Fibronektin | 97 | 10,2 |
| Osteopontin | 159 | 15,6 |
| Kollagen Typ I | 110 | 9 |
| Vitronektin | 107 | 5,2 |
| BoneSialoProtein | 109 | 6,6 |

| | **2. Versuch (menschliche Hüftknochenzellen) AP** | | | | | | | | | |
|---|---|---|---|---|---|---|---|---|---|---|
| | **A** | | **B** | | **C** | | **D** | | **E** | |
| | % Kon- trolle | STA +/- | % Kon- trolle | STA +/- | % Kon- trolle | STA +/- | % Kon- trolle | STA +/- | % Kon- trolle | STA +/- |
| Fibronektin | 99 | 6 | 100 | 1,5 | - | - | 84 | 2 | 102 | 5,3 |
| Osteopontin | 353 | 16,4 | 444 | 42 | 156 | 11,3 | 266 | 13 | 192 | 8,6 |
| Kollagen Typ I | 96 | 9 | 93 | 8,8 | - | - | 90 | 5,3 | 99 | 4 |
| Vitronektin | - | - | - | - | - | - | - | - | 101 | 8 |
| BoneSialoProtein | 168 | 12,8 | - | - | - | - | - | - | 97 | 4 |

| | **3. Versuch (menschliche Unterkieferknochenzellen)** | |
|---|---|---|
| | % Kontrolle | STA+/- |
| Fibronektin | 87 | 7 |
| Osteopontin | 305 | 22,9 |
| Kollagen Typ I | 100 | 7,8 |
| Vitronektin | - | - |
| BoneSialoProtein | 96 | 5,8 |

Als gesamter Mittelwert der alkalischen Phosphatasenaktivität über alle Ergebnisse der Zellkulturexperimente mit primär menschlichen Knochenzellen ergibt sich somit:

| | **Mittelwerte AP gesamt** | |
|---|---|---|
| | Mittelwerte Alkalische Phosphatase insgesamt | Mittelwerte |
| | (% Kontrolle) | (STA+/-) |
| Fibronektin | 94,8 | 5,3 |
| Osteopontin | 267,9 | 18,5 |
| Kollagen Typ I | 98,0 | 7,3 |
| Vitronektin | 104,0 | 6,6 |
| BoneSialoProtein | 117,5 | 7,3 |

Eine graphische Darstellung dieser Wert ist in Fig. 1 gezeigt.

Demnach führt bei allen humanen Knochenzellisolaten die Kultivierung der Knochenzellen auf den Osteopontin beschichteten Platten zur höchsten alkalischen Phosphatase-Sekretion in den Kulturüberständen.

### In-vitro-Mineralbildung

In einer zweiten Versuchsreihe wurde die In-vitro-Mineralbildung nach 8-tägiger Kultur primärer menschlicher Knochenzellen auf mit verschiedenen Knochenmatrixproteinen beschichteten Kulturplatten untersucht (Versuch 1 mit menschlichen Knochenzellen vom Oberschenkelknochen; Versuch 2 und 3 mit Knochenzellen vom menschlichen Unterkiefer).

| | **Ergebnisse der Zellkulturexperimente - Mineralisation** | | | | | |
|---|---|---|---|---|---|---|
| | **Versuch 1** | | **Versuch 2** | | **Versuch 3** | |
| | % Kontrolle | STA+/- | % Kontrolle | STA+/- | % Kontrolle | STA+/- |
| Fibronektin | 85 | 22,8 | 101 | 11,1 | 120 | 5,9 |
| Osteopontin | 99 | 9,2 | 108 | 16,3 | 107 | 7,6 |
| Kollagen Typ I | 101 | 31 | 100 | 6,5 | 83 | 5,2 |
| Vitronektin | 81 | 11,5 | 106 | 10,6 | 72 | 3,7 |
| BoneSialoProtein | 173 | 23,2 | 123 | 3,8 | 132 | 9,9 |

Wobei sich folgende Mittelwerte über die Versuche ergeben:

| **Ergebnisse Mittelwerte Mineralisation** | | |
|---|---|---|
| | Mittelwerte | Mittelwerte |
| | % in der Kontrolle | STA+/- |
| Fibronektin | 102,0 | 13,3 |
| Osteopontin | 104,7 | 11,0 |
| Kollagen Typ I | 94,7 | 14,2 |
| Vitronektin | 86,3 | 8,6 |
| BoneSialoProtein | 142,7 | 12,3 |

Eine graphische Darstellung dieser Wert ist in Fig. 2 gezeigt

In der Gesamtbetrachtung ergibt sich demnach, dass das Wachstum auf Kulturschalen mit Bone Sialoproteinbeschichtung einen konsistent signifikanten größeren positiven Effekt auf die Knochenmineralabbildung in vitro bei primären menschlichen Knochenzellen hat als andere getestete Substanzen.

Somit kann mit dem erfindungsgemäßen Verfahren zum Aufbereiten von Kollagenformkörpern sowie mit den aufbereiteten Kollagenformkörpern das Einwachsen von Knochenaugmentaten sowie die Knochenneubildung beziehungsweise das Wiedereinwachsen von Knochengewebe in leeren Zahnfächern nach der Zahnextraktion deutlich positiv beeinflusst werden.

## Patentansprüche

1. Verfahren zum Aufbereiten von Kollagenformkörpern
bei den Kollagenformkörper aus Kollagen Typ I und/oder Kollagen Typ III verwendet werden,
**dadurch gekennzeichnet,**
**dass** Knochenmatrixproteine, insbesondere Osteopontin oder Bone Sialoprotein in einer Flüssigkeit auf den Kollagenformkörper aufgebracht werden,
**dass** die Knochenmatrixproteine in der Flüssigkeit zwischen 0,01 Mass.-% bis 5 Mass.-% der Masse des Kollagenformkörpers aufweisen,
**dass** vor oder nach dem Aufbringen der Knochenmatrixproteine Prolinreste des Kollagens des Kollagenformkörpers jodiert werden,
**dass** der Kollagenformkörper zum Jodieren der Prolinreste in ein Medium, welches einen pH-Wert zwischen 5,0 und 6,0 bevorzugt um 5,6 hat und Natriumjodid sowie Wasserstoffperoxid aufweist und dort mit Lactoperoxidase versetzt wird, und
**dass** der Kollagenformkörper anschließend von nicht gebundenem Jod gereinigt wird.

2. Verfahren nach Anspruch 1,
**dadurch gekennzeichnet,**
**dass** die Knochenmatrixproteine in der Flüssigkeit zwischen 0,1 Mass.-% und 1 Mass.-% der Masse des Kollagenformkörpers aufweisen.

3. Verfahren nach Anspruch 1 oder 2,
**dadurch gekennzeichnet,**
**dass** der Kollagenformkörper in ein *Hydroxylapatit-Knochenzement* getränkt wird und dass der Kollagenformkörper anschließend getrocknet wird.

4. Verfahren nach einem der Ansprüche 1 bis 3,
**dadurch gekennzeichnet,**
**dass** auf den Kollagenformkörper ein *Hydroxylapatit-Knochenzement* aufgebracht wird und dass der Kollagenformkörper anschließend getrocknet wird.

5. Verfahren nach einem der Ansprüche 3 oder 4,
**dadurch gekennzeichnet,**
**dass** der *Hydroxylapatit-Knochenzement* vor dem Tränken oder Aufbringen mit Strontium versetzt wird.

6. Verfahren nach einem der Ansprüche 3 bis 5,
**dadurch gekennzeichnet,**
**dass** vor dem Tränken oder dem Aufbringen der *Hydroxylapatit-Knochenzement* mit Kollagenfasern versetzt wird.

7. Verfahren nach Anspruch 6,
**dadurch gekennzeichnet,**
**dass** die Kollagenfasern mit Knochenmatrixmolekülen angereichert sind.

8. Kollagenformkörper aufweisend Kollagen Typ I und/oder Kollagen Typ III,
**dadurch gekennzeichnet,**
**dass** der Kollagenformkörper 0,1 Mass.-% bis 5 Mass.-% Knochenmatrixproteine, insbesondere Osteopontin und/oder Bone Sialoprotein, aufweist,
**dass** etwa 2% der Prolinreste des Kollagens des Kollagenformkörpers jodiert sind, wobei der Kollagenformkörper zum Jodieren der Prolinreste in ein Medium, welches einen pH-Wert zwischen 5,0 und 6,0 bevorzugt um 5,6 hat und Natriumjodid sowie Wasserstoffperoxid aufweist und dort mit Lactoperoxidase versetzt wird, und der Kollagenformkörper anschließend von nicht gebundenem Jod gereinigt wird.

## Claims

1. Method for preparing shaped collagen bodies
in which shaped collagen bodies of collagen type I and/or collagen type III are used,
**characterized,**
**in that** bone matrix proteins, in particular osteopontin or bone sialoprotein, are applied to the shaped collagen body in a liquid,
**in that** the bone matrix proteins in the liquid comprise between 0.01 mass-% and 5 mass-% of the mass of the shaped collagen body,
**in that** before or after the application of the bone matrix proteins, proline residues of the collagen of the shaped collagen body are iodinated,
**in that** the shaped collagen body for iodination of the proline residues is placed in a medium which has a pH-value between 5.0 and 6.0, preferably around 5.6 and contains sodium iodide and hydrogen peroxide, and lactoperoxidase is added there, and
**in that** the shaped collagen body is then cleaned from unbound iodine.

2. Method according to claim 1,
**characterized,**
**in that** the bone matrix proteins in the liquid comprise between 0.1 mass-% and 1 mass-% of the mass of the shaped collagen body.

3. Method according to claim 1 or 2,
**characterized,**
**in that** the shaped collagen body is soaked in a hydroxyapatite bone cement and in that the collagen molding is then dried.

4. Method according to any one of claims 1 to 3,
**characterized,**
**in that** a hydroxyapatite bone cement is applied to the shaped collagen body and in that the shaped collagen body is then dried.

5. Method according to claim 3 or 4,
**characterized,**
**in that** strontium is added to the hydroxyapatite bone cement prior to soaking or application.

6. Method according to any one of claims 3 to 5,
**characterized,**
**in that** collagen fibers are added to the hydroxyapatite bone cement before it is soaked or applied.

7. Method according to claim 6,
**characterized,**
**in that** the collagen fibers are enriched with bone matrix molecules.

8. Shaped collagen body
comprising collagen type I and/or collagen type III,
**characterized,**
**in that** the shaped collagen body has 0.1 mass-% to 5 mass-% of bone matrix proteins, in particular osteopontin and/or bone sialoprotein,
**in that** about 2% of the proline residues of the collagen of the shaped collagen body are iodinated, wherein the shaped collagen body for iodination of the proline residues is placed in a medium which has a pH-value between 5.0 and 6.0, preferably around 5.6 and contains sodium iodide and hydrogen peroxide, and lactoperoxidase is added there, and the shaped collagen body is then cleaned from unbound iodine.

## Revendications

1. Procédé de préparation de corps moulés de collagène,
où des corps moulés de collagène composés du collagène de type 1 et/ou du collagène du type III sont utilisés,
**caractérisé en ce**
**que** des protéines de matrice osseuse, en particulier de l'ostéopontine ou de la sialoprotéine osseuse, sont appliquées dans un liquide sur le corps moulé de collagène, que les protéines de matrice osseuse présentent dans le liquide entre 0,01 % en masse à 5 % en masse de la masse du corps moulé de collagène,
**que** des résidus de proline du collagène du corps moulé de collagène sont iodés avant ou après l'application des protéines de matrice osseuse,
**que**, pour ioder les résidus de proline, le corps moulé de collagène est mélangé à de la lactoperoxydase, dans un milieu de pH compris entre 5,0 et 6,0, de manière préférée de 5,6, lequel milieu contient de l'iodure de sodium ainsi que du peroxyde d'hydrogène, et
**que** le corps moulé de collagène est ensuite nettoyé de l'iode qui n'a pas lié.

2. Procédé selon la revendication 1,
**caractérisé en ce**
**que** les protéines de matrice osseuse dans le liquide présentent entre 0,1 % en masse et 1 % en masse de la masse du corps moulé de collagène.

3. Procédé selon la revendication 1 ou 2,
**caractérisé en ce**
**que** le corps moulé de collagène est trempé dans un ciment osseux à base d'hydroxylapatite,
et **que** le corps moulé de collagène est ensuite séché.

4. Procédé selon l'une quelconque des revendications 1 à 3,
**caractérisé en ce**
**qu'**un ciment osseux à base d'hydroxylapatite est appliqué sur le corps moulé de collagène, et que le corps moulé de collagène est ensuite séché.

5. Procédé selon l'une quelconque des revendications 3 ou 4,
**caractérisé en ce**
**que** le ciment osseux d'hydroxylapatite est mélangé à du strontium avant d'être trempé ou appliqué.

6. Procédé selon l'une quelconque des revendications 3 à 5,
**caractérisé en ce**
**qu'**avant d'être trempé ou d'être appliqué, le ciment osseux à base d'hydroxylapatite est mélangé à des fibres de collagène.

7. Procédé selon la revendication 6,
**caractérisé en ce**
**que** les fibres de collagène sont enrichies en molécules de matrice osseuse.

8. Corps moulé de collagène,
présentant du collagène de type 1 et/ou du collagène de type III,
**caractérisé en ce**
**que** le corps moulé de collagène présente 0,1 % de masse à 5 % de masse de protéines de matrice osseuse, en particulier de l'ostéopontine et/ou de la sialoprotéine osseuse, qu'environ 2 % des résidus de proline du collagène du corps moulé de collagène sont iodés, dans lequel pour ioder les résidus de proline le corps moulé de collagène est mélangé à de la lactoperoxydase dans un milieu de pH compris entre 5,0 et 6,0, de manière préférée de 5,6, le milieu comprenant de l'iodure de sodium ainsi que du peroxyde d'hydrogène ; et le corps moulé de collagène est ensuite nettoyé avec de l'iode non lié.
